(19) Europäisches Patentamt
European Patent Office
Office européen des brevets

(11) Veröffentlichungsnummer: **0 149 054**
**B1**

(12) # EUROPÄISCHE PATENTSCHRIFT

(45) Veröffentlichungstag der Patentschrift: **04.02.87**

(51) Int. Cl.⁴: **C 07 C 121/00**, C 11 B 9/00

(21) Anmeldenummer: **84113981.9**

(22) Anmeldetag: **19.11.84**

(54) **Verfahren zur Herstellung von Cyclododecenylacetonitril und seine Verwendung als Riechstoff oder Riechstoffkomponente.**

(30) Priorität: **11.01.84 DE 3400689**

(43) Veröffentlichungstag der Anmeldung: **24.07.85 Patentblatt 85/30**

(45) Bekanntmachung des Hinweises auf die Patenterteilung: **04.02.87 Patentblatt 87/6**

(84) Benannte Vertragsstaaten: **AT BE CH DE FR GB IT LI NL**

(73) Patentinhaber: **HÜLS AKTIENGESELLSCHAFT, - RSP Patente / PB 15 - Postfach 13 20, D-4370 Marl 1 (DE)**

(72) Erfinder: **Kaufhold, Manfred, Dr., Jasminweg 20, D-4370 Marl (DE)**
Erfinder: **Slagmulder, André, 39, Avenue Claude Sommer, F-95250 Beauchamp (FR)**

(56) Entgegenhaltungen:
**BULLETIN OF THE CHEMICAL SOCIETY OF JAPAN, Band 53, Nr. 10, Oktober 1980, Seiten 2895-2904, The Chemical Society of Japan, JP; T. MASAMUNE u.a.: "Steric effect in regioselective cyclization of 3,4-epoxy alcohols to oxetanes"**
**CHEMICAL ABSTRACTS, Band 71, Nr. 3, 21. Juli 1969, Seite 280, Nr. 12637s, Columbus, Ohio, US; NOBUO ITOH u.a.: "Nature (endo or exo) of the double bond in the condensation products of cyclic ketones with cyanoacetate or cyano-acetic acid"**
**R. ADAMS u.a.: "Organic Reactions", Band 15, 1967, Seiten 204-599, Kapitel 2, G. JONES: "The Knoevenagel condensation", John Wiley & Sons, Inc., New York, US;**
**HELVETICA CHIMICA ACTA, Band 33, Fasc. 6, Nr. 186-187, 16. Oktober 1950, Seiten 1437-1448; O. SCHNIDER u.a.: "Synthese von Morphinanen"**
**THE JOURNAL OF ORGANIC CHEMISTRY, Band 44,**

(56) Entgegenhaltungen: (Fortsetzung)
**Nr. 25, 7. Dezember 1979, Seiten 4640-4649, American Chemical Society, US; STEPHEN A. DiBIASE u.a.: "Direct synthesis of alpha,beta-unsaturated nitriles from acetonitrile and carbonyl compounds: survey, crown effects, and experimental conditions"**
**SYNTHESIS, INTERNATIONAL JOURNAL OF METHODS IN SYNTHETIC ORGANIC CHEMISTRY, Nr. 9, September 1977, Seiten 629-632; STEPHEN A. DiBIASE u.a.: "A convenient synthesis of aliphatic alpha,beta-unsaturated nitriles from acetonitrile"**
**HELVETICA CHIMICA ACTA, Band 62, Fasc. 3, Nr. 92, 20. April 1979, Seiten 882-893; A. FISCHLI: "Cob(I)alamin als Katalysator 4. Mitteilung. Reduktion von alpha,beta-ungesättigten Nitrilen"**

**EP 0 149 054 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Cyclododecenylacetonitril kann man durch Kondensation von Cyclododecanon mit Acetonitril in Gegenwart von stark basischen Katalysatoren herstellen (Stephen A. Di Biase et al., Journal of Org. Chem. Vol. 44, No 25 (1979) S. 4640). Man erhält ein Gemisch (3) der Isomeren (1) und (2), jedoch nur in Ausbeuten von ca. 45 %.

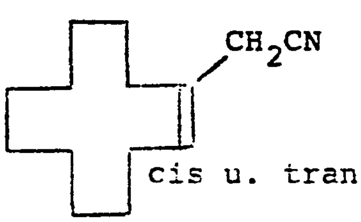
cis u. trans

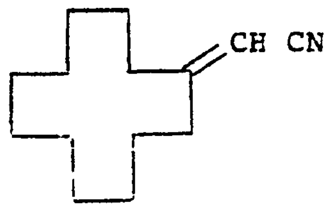

Gemisch von 1 und 2

1 2 3

Diese geringe Ausbeute ist für eine technische Verwertung völlig unzureichend. Hieraus ergab sich die Aufgabe, ein einfaches und wirtschaftliches Verfahren zur Herstellung eines Gemisches von Cyclododecenylacetonitril (1) und (2) zu finden.

Diese Aufgabe wird erfindungsgemäß entsprechend den Angaben der Patentansprüche gelöst.

Durch Umsetzung von Cyclododecanon mit Cyanessigsäure in Gegenwart eines üblichen KNOEVENAGEL-Katalysators, wie Ammoniumacetat, Piperidin, β-Alanin und andere Amine in Gegenwart von Eisessig erhält man das Cyclododecenylacetonitril-Gemisch (3) in über 70 %iger Ausbeute. Ammoniumacetat hat den Vorteil, daß es sehr preiswert ist, deshalb nicht zurückgewonnen werden muß und leicht von den Reaktionsprodukten abtrennbar ist.

In den Lehrbüchern der Organischen Chemie werden bei diesem Reaktionstyp - bekannt unter der Bezeichnung KNOEVENAGEL-Reaktion - nur dann gute Ausbeuten genannt, wenn kurzkettige Ketone oder durch einen aromatischen Ring aktivierte Carbonylverbindung eingesetzt werden. In der Reihe der Ketone, Aceton, Butanon und Cyclohexanon sinkt die Ausbeute von 90 über 80 auf 70 %, d. h. mit wachsender Kettenlänge nimmt die Ausbeute stark ab (s. ORGANIKUM, 12. Aufl., VEB Deutscher Verlag der Wissenschaften, Berlin, 1973, Seite 508).

Es war also nicht zu erwarten, daß diese Reaktion bei Ringsystemen mit der im Vergleich zum Cyclohexanon doppelten C-Zahl wirtschaftlich interessante Ausbeuten liefert, insbesondere nicht, daß ein Keton mit 12 C-Atomen mit Cyanessigsäure und einem sehr schwach basischen Katalysator (im Gegensatz zu der Methode von Stephen A. DiBiase et al., s. o.) mit Cyanessigsäure in über 70 %iger Ausbeute das entsprechend substituierte Acetonitril ergibt.

Man setzt Cyclododecanon mit Cyanessigsäure im Molverhältnis von 5: 1 bis 1: 5, vorzugsweise von 1: 2 bis 1: 1 um in Gegenwart von 5 bis 1, vorzugsweise 3 bis 2 Gewichtsprozent eines üblichen KNOEVENAGEL-Katalysators, insbesondere Ammoniumacetat, bezogen auf Cyclododecenon. Die Umsetzung erfolgt bei 100 bis 170°C, vorzugsweise 120 bis 140°C. Das erhaltene Reaktionsprodukt reinigt man destillativ bei 1 bis 50 mbar. Man erhält das Cyclododecenylacetonitril in einer Reinheit von über 99 %. Es hat einen Geruch nach Ammoniak. Nach dem Durchleiten eines Inertgases, wie z. B. Stickstoff, beispielsweise 50 bis 200 1 Stickstoff durch 1 bis 5 1 des Gemisches der Cyclododecenylacetonitril-Isomeren (3) während einer Zeit von 0,5 bis 5, vorzugsweise 1 bis 3 Stunden bei 10 bis 100 °C, vorzugsweise 15 bis 30 °C ist der Geruch nach Ammoniak vollständig verschwunden.

Nach dieser Behandlung hat das reine Gemische (3) von Cyclododecenylacetonitril (1) und (2) einen unerwarteten angenehmen an Rosen und Maiglöckchen erinnernden Geruch, der zudem noch eine Moschusnote enthält. Das Gemisch (3) der reinen Isomeren (1) und (2) zeigten eine hervorragende Riechstoffqualität und hat auch als Riechstoffkomponente unerwartet gute Eigenschaften.

Bisher gab es keinen Hinweis, daß diese Stoffe in reiner Form eine gute Riechstoff-Qualität haben.

Es besteht ein großes Interesse an Riechstoffen, die leicht und wirtschaftlich herstellbar sind, große chemische Beständigkeit zeigen und ein breites Spektrum an Anwendungsmöglichkeiten bei unterschiedlichen Riechstoffkompositionen besitzen.

Diese Eigenschaften hat das Gemisch (3) von 1 und 2 in überraschend hohem Maß.

Nach dem erfindungsgemäßen Verfahren erhält man die neuen Riechstoffe in einfacher und wirtschaftlicher Weise in guter Ausbeute.

Im Vergleich zu vielen anderen Riechstoffen wie z. B. Estern und Aldehyden zeigt das Cyclododecenylacetonitril eine hohe Beständigkeit gegen Chemikalien und ist in dem pH-Bereich von ca. 3 bis 14 stabil. Besonders seine unerwartet hohe Alkalibeständigkeit macht es geeignet zur Parfümierung von Waschmitteln, Seifen usw.

Mit anderen Stoffen, vornehmlich mit anderen Riechstoffen, kann (3) in den verschiedensten Mengenverhältnissen zu neuen Riechstoffkompositionen vermischt werden. Dabei beträgt der Anteil des bean-Cyclododecenylacetonitrils (3) im allgemeinen bis zu 60, vorzugsweise 1 bis 50 Gewichtsprozent. Durch den Zusatz erhalten die Kompositionen einen abgerundeten Geruch, ohne dadurch ihre interessante, charakteristische Geruchsnote zu verlieren. Es ist überraschend, daß dieser Effekt bei kompositionen und "Basen" mit sehr unterschiedlichen Geruchscharakteren erzielt wird (siehe Beispiele).

Die das Cyclododecenylacetonitril, enthaltenen Riechstoffkompositionen können als Parfüm oder zur Parfümierung von Kosmetika, wie z. B. Cremes, Feinseifen und Lotionen dienen. Außerdem können sie zur Geruchsverbesserung technischer Produkte, wie z. B. Wasch- und Reinigungsmittel, Desinfektionsmittel und Textilhilfsmittel verwendet werden.

Die nachfolgenden Beispiele sollen den Gegenstand der vorliegenden Erfindung näher erläutern.

**Beispiel 1**

**Synthese des Cyclodecenylacetonitrils**

In einer Rührapparatur, die mit Thermometer, Rückflußkühler und Wasserauskreiser ausgestattet ist, werden folgende Produkte eingesetzt:

434 g,(= 5 Mol) Cyanessigsäure
910 g (= 5 Mol) Cyclododecanon
650 g Toluol
21 g Ammoniumacetat

Bei 128 bis 130 °C wird dieses Gemisch unter Rühren und Stickstoffabdeckung 30 Stunden lang am Rückfluß zum Sieden erhitzt und dabei das sich bildende Wasser, 80 g, ausgekreist. Hierbei entsteht ein Kohlendioxid-Strom.

Auswaage: 1 667 g

Dieser Austrag wird an einer Brücke, die sich auf einer 10 cm langen mit 10 x 10 mm Glasraschigringen gefüllten Glaskolonne befindet, destilliert, wobei zunächst bei Normaldruck Toluol, 480 g, abdestilliert wird. In einem Siedebereich von 120 bis 180 °C bei 18 bis 20 mbar wird vom Rückstand, 89 g, abgetoppt,

Dieser Destillatanfall, 996 g, wird an einer 0,5 m langen mit Multifil-Füllkörpern gefüllten Glaskolonne fraktioniert. Bei 13 mbar werden 729 g Destillat in einem Siedebereich von 70 bis 179 °C erhalten.

Die gaschromatographisch ermittelte Reinheit des Isomerengemisches liegt bei 99,1 %; die Gehalte der einzelnen Komponenten waren:

1. Komponente 63,2 %
2. Komponente 32,0 %
3. Komponente 3,9 %

Die Komponenten 1 und 2 sind der Struktur 1 zuzuordnen und sind trans-cis Isomere. Die 3. Komponente hat wahrscheinlich die Struktur 2.

Die Ausbeute an (3) errechnet sich zu 71,1 %, bezogen auf den Einsatz. Das erhaltene Produkt riecht nach Ammoniak. Durch 1 bis 5 1 von (3) leitet man ca. 50 bis 200 1 Stickstoff pro Stunde durch ein in das Produkt eintauchendes am Ende verengtes Rohr oder durch ein Rohr, an dessen Ende sich eine Fritte befindet. Die Temperatur des Produktes liegt bei 15 bis 30 °C, d. h. Raumtemperatur oder höher bis etwa 100 °C. Je nach Temperatur werden zeiten von 0,5 bis 5 Stunden benötigt. Das Ende des Durchleitens ist an der deutlichen Geruchsverbesserung leicht feststellbar.

Man erhält ein sehr reines Gemisch der Isomeren des Cyclododecenylacetonitrils mit einem angenehmen an Rose und Maiglöckchen erinnernden Geruch, der auch noch eine Moschusnote enthält.

**Beispiel 2**

Eine blumige "Maiglöckchen-Komposition" wird durch Mischen folgender Komponenten hergestellt:

25 Teile Jasmin-Base
3 Teile Phenylethyl-isobutyrat
5 Teile Geraniol 98 = 2-trans-3,7-Dimethyl-2,6-octadien-8-ol (Handelsprodukt der Fa. BBA)
5 Teile Citronnellol = 3,7-Dimethyl-6-octen-1-ol (Handelsprodukt der Fa. BBA)
1 Teil Zimtsäure-methylester 1 %
10 Teile β-Phenylethanol
30 Teile Lylial = p-tert.-Butyl-α-methyl-phenylpropionaldehyd (Handelsprodukt der Fa. GIVAUDAN SA)
5 Teile Cyclamen-Aldehyd = p-Isopropyl-α-methyl-phenylpropionaldehyd
9 Teile Linalool-synthetisch = 3.7-Dimethyl-1.6-octadien-3-ol (Handelsprodukt der Fa. GIVAUDAN SA)
93 Teile Summe

Diese Komposition besitzt den typischen Maiglöckchengeruch.

Ein Zusatz von 32 Teilen Cyclododecenylacetonitril ergibt 125 Teile einer neuartigen Duftkomposition, die

nicht nur die blumige Maiglöckchen-Note zeigt, sondern ein volleres abgerundetes Geruchsspektrum mit zum Teil herberem, würzigem Charakter besitzt.

**Beispiel 3**

Eine sogenannte VIOLETTE-Komposition wird durch Mischen folgender Komponenten hergestellt:
480 Teile Vestinol C = o-Phthalsäure-di-n-butylester (Handelsprodukt der Fa. CWH)
25 Teile Cédrol,s. S. Arctander, perfume and Flavor Chemicals Montclair N.J. (USA) 1969, Nr. 598 (Handelsprodukt der Pa. MANE FILS)
20 Teile Base Vert de Violette (Handelsprodukt der Fa. E.R.P.)
175 Teile Colophane (Handelsprodukt der Fa. D.R.T.)
50 Teile Ionone Savon (Handelsprodukt der Pa. SORDES)
100 Teile Terpens d'Isorone (Handelsprodukt der Fa. SORDES)
25 Teile Benzoesäure-benzylester
50 Teile Bois de Cédre de Virgine (Handelsprodukt der Pa. ADRIEN)
25 Teile Benzylacetat
50 Teile Terpinéol (Handelsprodukt der Fa. D.R.T.)
100 Teile Summe
Diese Komposition besitzt einen holzigen, etwas flachen, an Baumharze erinnernden Geruch.
Ein Zusatz von 50 Teilen Cyclododecenylacetonitril ergibt 150 Teile einer neuartigen Duftkomposition mit einem gegenüber der o. g. Komposition abgerundeteren Geruch, der eine volle warme Komponente enthält.

**Beispiel 4**

Eine sogenannte SANTAL-Base-Komposition wird durch Mischen folgender Komponenten hergestellt:
36 Teile Isobornyl-Cyclohexanol
29,8 Teile Dicydol = 3(4), 8(9)-Dihydroxy-methyl-tri-cyclo $[5.2.1^{2-6}]$ decan (Handelsprodukt der Fa. RUHRCHEMIE)
9 Teile Methylcyclododecylether (10 %ig)
3 Teile Arova = 1,4-Dioxa-cyclo-hexadecan-5.16-dion CXBM (Handelsprodukt der Fa. E.R.p.)
0,2 Teile prunolide (10 %ig) (Handelsprodukt der Fa. GIVAUDAN)
2 Teile Formiat des trans-β-Decyhydronaphthols
80 Teile Summe
Diese Komposition besitzt einen süßlichen, moschusartigen, an Sandelholz erinnernden Geruch.
Ein Zusatz von 20 Teilen Cyclododecenylacetonitril ergibt 100 Teile einer nevartigen (Geruchs-)Base mit einem gegenüber der Ausgangsmischung abgerundeteren, wärmeren, milderen Geruch, der eine etwas blumigere Note hat.

**Beispiel 5**

Eine spezielle, sogenannte PATCHOULY-Base-Komposition wird durch Mischen folgender Komponenten hergestellt:
5 Teile Isobornyl-Cyclohexanol
5 Teile Dicydol (s. Beispiel 4)
17 Teile Methylcyclododecylether (10 %ig)
1 Teil Formiat des trans-β-Decahydronaphthols
10 Teile Formiat des p-tert.-Butylcyclohexanols
10 Teile Vertofix coeur (Handelsprodukt der Pa. IPP)
1 Teil p-tert.-Butylcyclohexanol
5 Teile Vetchouly (Handelsprodukt der Pa. BERJE)
20 Teile Bomme Copahn (Handelsprodukt der Pa. ADRIEN)
10 Teile Bomme Gurjum (von derselben Firma)
5 Teile Acetat des Cédrényle,S. Arctander, Perfume and Plavor Chemicals Montclair N.J. (USA) 1969, Nr. 597 (Handelsprodukt der Pa. IPP)
1 Teil Isocyclocitral,S. Arctander, Perfume and Plavor Chemicals Montclair N.J. (USA) 1969, Nr. 761 (Handelsprodukt derselben Pirma)
2 Teile Perou Bomme (Handelsprodukt der Pa. ADRIEN)
0,25 Teile Mélange PM 531/PM 517 á 2 % (Pyrazines) (Handelsprodukt der Pa. BBA)
3 Teile Pormaldehyd-di-cyclododecylacetal

2,75 Teile Cyclododecenon
98 Teile Summe
Diese Komposition besitzt den charakteristischen Patchouly-Geruch.

Ein Zusatz von 27 Teilen Cyclododecenylacetonitril ergibt 125 Teile einer neuartigen (Geruchs-)Base, bei der der Patchouly-Geruch erhalten ist, die aber einen abgerundeten Geruch mit einer warmen, erfrischenden Note aufweist.

## Patentansprüche

1. Verfahren zur Herstellung von Cyclododecenylacetonitril dadurch gekennzeichnet,
daß man Cyclododecanon mit Cyanessigsäure im Molverhältnis von 5: 1 bis 1: 5 bei Temperaturen von 100 bis 170 °C in Gegenwart von 5 bis 1 Gewichtsprozent eines üblichen KNOEVENAGEL-Katalysators, bezogen auf Cyclododecanon, umsetzt, das erhaltene Reaktionsprodukt bei 1 bis 50 mbar destilliert und anschließend während einer Zeit von 0,5 bis 5 Stunden bei 10 bis 100 °C ein Inertgas durchleitet.

2. Verfahren nach Anspruch 1,
dadurch gekennzeichnet,
daß man als KNOEVENAGEL-Katalysator Ammoniumacetat einsetzt.

3. Verfahren nach Anspruch 1 und 2,
dadurch gekennzeichnet,
daß man ein Molverhältnis vom 1: 2 bis 1: 1 einsetzt.

4. Verfahren nach Anspruch 1 bis 3,
dadurch gekennzeichnet,
daß man die Umsetzung bei einer Temperatur von 120 bis 140 °C vornimmt.

5. Verfahren nach Anspruch 1 bis 4,
dadurch gekennzeichnet,
daß man 3 bis 2 Gewichtsprozent Ammoniumacetat einsetzt.

6. Verfahren nach Anspruch 1 bis 5,
dadurch gekennzeichnet,
daß man durch 1 bis 5 1 des Reaktionsproduktes 50 bis 200 1 Stickstoff/Stunde während einer Zeit von 1 bis 3 Stunden bei 15 bis 30 °C durchleitet.

7. Verwendung eines Cyclododecenylacetonitril-Isomerengemisches als Riechstoff oder als Riechstoffkomponente.

## Claims

1. A process for the production of cyclododecenylacetonitrile, characterised in that cyclododecanone is reacted with cyanoacetic acid in a molar ratio of from 5:1 to 1;5 at a temperature of from 100 to 170°C in the presence of from 5 to 1% by weight of a conventional Knoevenagel catalyst, based on the cyclododecanone, the resuling reaction product is distilled at 1 to 50 mbar and subsequently an inert gas is pessed through it at 10 to 100°C over a period of 0.5 to 5 hours.

2. A process according to claim 1, characterised in that ammonium acetete is introduced as the Knoevenagel catalyst.

3. A process according to claim 1 or 2, characterised in that a molar ratio of from 1:2 to 1:1 is employed.

4. A process according to any of claims 1 to 3, characterised in that the reaction is carried out at a temperature of from 120 to 140°C.

5. A process according to any of claims 1 to 4, characterised in that from 3 to 2% by weight of ammonium acetate is introduced.

6. A process according to any of claims 1 to 5, characterised in that 50 to 200 litres of nitrogen per hour are passed through 1 to 5 litres of the rection product at 15 to 30°C over a period of 1 to 3 hours.

7. The use of a cyclododecenyl-acetonitrile isomer mixture as an odorant or an odour-imparting component.

## Revendications

1. Procédé de préparation de cyclodécényl-acétonitrile,
caractérisé par le fait que l'on fait réagir la cyclo-dodécanone sur l'acide cyanacétique dans un rapport molaire de 5 : 1 à 1 : 5, à des températures de 100 à 170°C, en présence de 5 à 1 % en poids d'un catalyseurs "KNOEVENAGEL" usuel, relativement à la cyclo-dodécanone, que l'on distille le produit de réaction obtenir sous 1 à 50 mbar et qu'on fait ensuit passer un gaz inerte au travers pendant un temps de 0,5 à 5 heures, à une température de 10 à 100°C.

2. Procédé selon la revendication 1,
caractérisé par le fait qu'on utilise, comme catalyseur "KNOEVENAGEL", de l'acétate d'ammonium.
3. Procédé selon les revendications 1 et 2,
caractérisé par le fait que l'on utilise un rapport molaire de 1 : 2 à 1 : 1.
4. Procédé selon les revendications 1 à 3,
caractérisé par le fait que l'on effectue la réaction à une temperature de 120 à 140° C.
5. Procédé selon les revendications 1 à 4,
caractérisé par le fait que l'on utilise de 3 à 2 % en poids d'acétate d'ammonium.
6. Procédé selon les revendications 1 à 5,
caractérisé par le fait qu'on fait passer, à travers 1 à 5 litres du produit de réaction, de 50 à 200 litres d'azote par heure, pendant un temps de 1 à 3 heures, à une température de 15 à 30° C.
7. L'utilisation d'un mélange de cyclo-dodécénylacétonitriles isoméres comme parfum ou comme constituant de parfum.